(19) Europäisches Patentamt
European Patent Office
Office européen des brevets

(11) Veröffentlichungsnummer: **0 297 380 B1**

(12) **EUROPÄISCHE PATENTSCHRIFT**

(45) Veröffentlichungstag der Patentschrift: **05.10.94**

(51) Int. Cl.5: **C07D 403/12**, C07D 209/42, C07D 209/34, C07D 209/12, A61K 31/40

(21) Anmeldenummer: **88109770.3**

(22) Anmeldetag: **20.06.88**

Die Akte enthält technische Angaben, die nach dem Eingang der Anmeldung eingereicht wurden und die nicht in dieser Patentschrift enthalten sind.

(54) **Indolylpropanole, Verfahren zu ihrer Herstellung und ihre Verwendung sowie die Verbindungen enthaltende Zubereitungen.**

(30) Priorität: **27.06.87 DE 3721260**

(43) Veröffentlichungstag der Anmeldung:
**04.01.89 Patentblatt 89/01**

(45) Bekanntmachung des Hinweises auf die Patenterteilung:
**05.10.94 Patentblatt 94/40**

(84) Benannte Vertragsstaaten:
**AT BE CH DE ES FR GB IT LI NL SE**

(56) Entgegenhaltungen:
**FR-A- 2 567 885**

(73) Patentinhaber: **Beiersdorf-Lilly GmbH**
**Wiesingerweg 25**
**D-20253 Hamburg (DE)**

(72) Erfinder: **Stenzel, Wolfgang, Dr.**
**Lerchenweg 8**
**D-2057 Reinbek (DE)**
Erfinder: **Armah, Ben, Dr.**
**Hemmingstedter Weg 123f**
**D-2000 Hamburg 52 (DE)**
Erfinder: **Beuttler, Thomas, Dr.**
**Hans-Lange-Strasse 8**
**D-2000 Hamburg 55 (DE)**

(74) Vertreter: **Hudson, Christopher Mark et al**
**Erl Wood Manor**
**Windlesham Surrey GU20 6PH (GB)**

EP 0 297 380 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind neue substituierte Indolylpropanole der Formel I

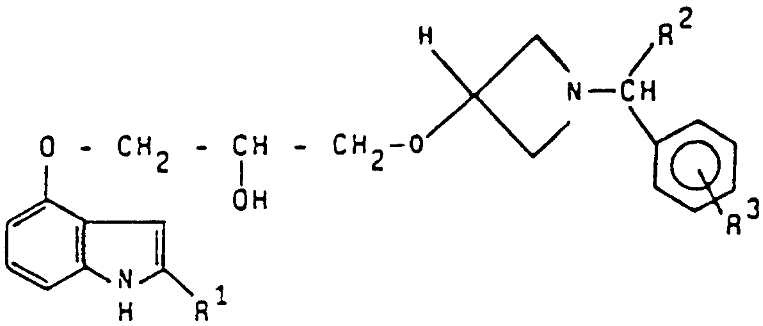

in der
$R^1$ eine Cyano-, Carboxamid-, Alkoxycarbonyl-, Hydroxyl- oder Acetylgruppe bedeutet,
$R^2$ Pyridinyl, Thienyl, Phenyl oder substituiertes Phenyl bedeutet, das durch Halogen, Alkyl, Cycloalkyl, Alkoxy, Difluormethoxy, Difluormethylthio, Trifluormethoxy, Trifluormethylthio, Trifluorethoxy, Alkoxyalkoxy, Cycloalkylalkoxy, Alkoxyalkyl, Alkylthioalkoxy, Alkylsulfinylalkoxy, Alkylsulfonylalkoxy, Alkoxyalkylthio, Alkoxyalkylsulfinyl, Alkoxyalkylsulfonyl, Alkylthioalkyl, Alkylsulfinylalkyl, Alkylsulfonylalkyl und Dialkylaminoalkoxy mono- oder disubstituiert ist, und
$R^3$ Wasserstoff, Alkoxy, Halogen, Difluormethoxy, Difluormethylthio, Trifluormethoxy, Trifluormethylthio, Trifluorethoxy, Alkoxyalkoxy, Alkoxyalkyl, Alkylthioalkoxy, Alkylsulfinylalkoxy, Alkylsulfonylalkoxy, Alkoxyalkylthio, Alkoxyalkylsulfinyl, Alkoxyalkylsulfonyl, Alkylthioalkyl, Alkylsulfinylalkyl, Alkylsulfonylalkyl oder Dialkylaminoalkoxy bedeutet,
oder ein physiologisch verträgliches hydrolysierbares Derivat davon, in dem die Hydroxygruppe in 2-Stellung der 3-Aminopropoxy-Seitenkette in veresterter Form vorliegt, sowie ihre tautomeren Formen und ihre Salze sowie Säureadditionssalze, wobei in allen vorstehend genannten Fällen Alkyl der Alkylgruppen oder von Alkylteilen oder Alkylenteilen von Gruppen jeweils geradkettiges oder verzweigtes Alkyl beziehungsweise Alkylen mit 1 bis 6 Kohlenstoffatomen bedeutet und Cycloalkyl 3 bis 7 Kohlenstoffatome besitzt, sowie Verfahren zu ihrer Herstellung und ihre Verwendung und Zubereitungen, die diese Verbindungen enthalten.

Physiologisch hydrolysierbare Derivate sind Derivate, die unter physiologischen Bedingungen zu den entsprechenden Verbindungen gespalten werden, die eine Hydroxygruppe in 2-Stellung der Propoxy-Seitenkette aufweisen.

Eine Gruppe von solchen Derivaten in veresterter Form der Verbindungen der Formel I besteht z.B. aus den Verbindungen der Formel Ia

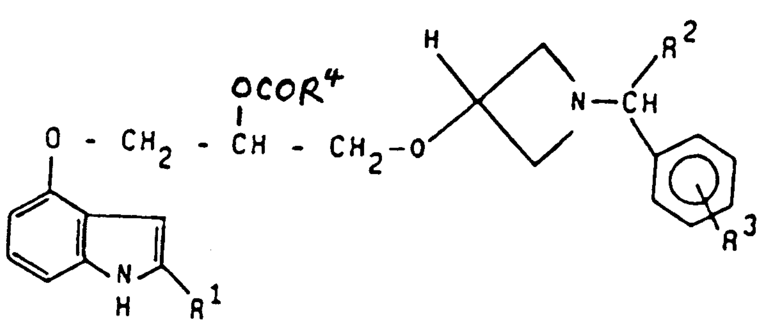

(Ia)

worin $R^1$, $R^2$, $R^3$ und Y die obengenannte Bedeutung besitzen, und
$R^4$ für Alkyl mit 1 bis 12 Kohlenstoffatomen, Cycloalkyl mit 3 bis 7 Kohlenstoffatomen, Phenyl, Phenylalkyl mit 7 bis 12 Kohlenstoffatomen, im Phenylring durch Alkyl mit 1 bis 4 Kohlenstoffatomen monosubstituiertes Phenyl oder Phenylalkyl mit 7 bis 12 Kohlenstoffatomen, im Phenylring durch Halogen mit einer Ordnungszahl von 9 bis 35 mono- oder gleich oder verschieden disubstituiertes Phenyl oder Phenylalkyl mit 7 bis 12 Kohlenstoffatomen, oder im Phenylring durch Alkoxy mit 1 bis 4 Kohlenstoffatomen mono- oder gleich oder verschieden di- oder gleich oder verschieden trisubstituiertes Phenyl oder Phenylalkyl mit 7 bis 12 Kohlenstoffatomen steht.

Bevorzugt sind die Verbindungen der Formel I, in denen die Hydroxygruppe in 2-Stellung der Propoxy-Seitenkette in unveresterter Form vorliegt.

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen. Insbesondere wird die Oxindolform der in 2-Stellung durch Hydroxy substituierten Indolgruppe umfaßt.

Obgleich pharmazeutisch verträgliche Salze und Säureadditionssalze der neuen Verbindungen der Formeln I und Ia und deren tautomere Formen bevorzugt sind, liegen alle Salze innerhalb des Bereichs der Erfindung. Alle Salze sind wertvoll zur Herstellung der Verbindungen, selbst wenn das spezielle Salz nur als Zwischenprodukt gewünscht wird, wie zum Beispiel, wenn das Salz nur für Zwecke der Reinigung oder Identifizierung gebildet wird, oder wenn es als ein Zwischenprodukt bei der Herstellung eines pharmazeutisch verträglichen Salzes, beispielsweise durch Ionenaustauschverfahrensweisen, verwendet wird.

Die Verbindungen der allgemeinen Formel I und deren Salze enthalten asymmetrische Kohlenstoffatome. Daher sind auch die verschiedenen optischen Isomeren sowie die Diastereoisomeren Gegenstand der Erfindung ebenso wie die Salze und Additionssalze dieser Verbindungen mit Säuren. Die Racemate können nach an sich bekannten Methoden in ihre optischen Antipoden aufgetrennt werden.

Bevorzugt sind Verbindungen, in denen das asymmetrische Kohlenstoffatom $C^{(1)*}$ im Propanolaminteil der Formel I die S-Konfiguration besitzt.

Mit den Verbindungen der vorliegenden Erfindung strukturell verwandte Verbindungen sind in der europäischen Patentschrift Nr. 25 111 und der deutschen Offenlegungsschrift 35 24 955 beschrieben. Die Verbindungen der vorliegenden Erfindung werden aber von diesen Offenbarungen weder spezifisch offenbart noch nahegelegt.

Soweit nicht anders angegeben, können die erfindungsgemäßen Alkylgruppen und Alkylteile beziehungsweise Alkylenteile von Gruppen geradkettig oder verzweigt sein und sie besitzen jeweils 1 bis 6 Kohlenstoffatome, vorzugsweise 1 bis 4 Kohlenstoffatome, insbesondere 1 oder 2 Kohlenstoffatome. Beispiele für solche einmal oder mehrfach Alkyl oder Alkylen enthaltende Gruppen sind Alkoxy, Alkoxyalkoxy, Alkoxyalkyl, Alkylthioalkoxy, Alkoxyalkylthio oder Dialkylalkoxy. Bevorzugte Alkyl- oder Alkenteile sind Methyl, Ethyl, n-Propyl, Isopropyl, Butyl beziehungsweise entsprechend Methylen, Ethylen, n- oder Isopropylen und Butylen.

Erfindungsgemäße Cycloalkylalkoxygruppen, z.B. $R^2$, besitzen 4 bis 13 Kohlenstoffatome, insbesondere 4 bis 9 Kohlenstoffatome.

Die Alkoxyreste der Cycloalkylalkoxygruppen haben 1 bis 6 Kohlenstoffatome, bevorzugt werden Methylen, Ethylen und Propylen als Alkylenreste der Alkoxyreste.

Erfindungsgemäße Cycloalkylgruppen, z.B. $R^2$, und -teile wie Cycloalkylreste von Cycloalkylalkoxygruppen besitzen 3 bis 7 Kohlenstoffatome, insbesondere 3 bis 6 Kohlenstoffatome. Besonders bevorzugt sind Cyclopropyl und Cyclohexyl.

Halogenreste $R^2$ und $R^3$ sind vorzugsweise F, Cl, Br, insbesondere F oder Cl. Alkyl oder Alkylteil der Alkoxygruppe des Substituenten $R^2$ und $R^3$ sind $C_{1-6}$-Alkyl, vorzugsweise Methyl, Ethyl, n- und Isopropyl und Butyl.

Bevorzugte Reste $R^3$ sind Wasserstoff, Alkoxy und Halogen, insbesondere aber Wasserstoff.

Bevorzugte Alkoxygruppen der Alkoxycarbonylgruppe $R^1$ besitzen 1 bis 6, vorzugsweise 1 bis 4, Kohlenstoffatome. Besonders bevorzugt sind Methoxy und Ethoxygruppen. Vorzugsweise ist $R^1$ Cyano.

Trifluorethoxyreste von $R^2$ oder $R^3$ sind vorzugsweise $F_3CCH_2O$-Reste.

Pyridinyl ist vorzugsweise Pyridin-4-yl und Thienyl ist vorzugsweise Thien-3-yl.

Vorzugsweise ist $R^2$ Phenyl oder substituiertes Phenyl, insbesondere aber Phenyl. Die Phenylgruppe kann eine oder zwei der genannten Substituenten tragen, die gleich oder verschieden sein können. Sind die Phenylgruppen disubstituiert, dann sind die Substituenten vorzugsweise gleich.

Substituierte Phenylgruppen $R^2$ sind mit den angegebenen Substituenten vorzugsweise in 3- und/oder 4-Stellung substituiert, insbesondere in 4-Stellung monosubstituiert. Ebenso befindet sich der Rest $R^3$ vorzugsweise in der 3- oder 4-Stellung, insbesondere in der 4-Stellung.

Vorzugsweise ist $R^3$ Wasserstoff. Besonders bevorzugt werden Verbindungen der Formel I oder Ia in der $R^3$ Wasserstoff bedeutet und die übrigen Reste die angegebene Bedeutung haben, insbesondere aber ist dann $R^2$ Phenyl.

Alkoxyalkoxy-, Alkoxyalkyl-, Alkylthioalkoxy-, Alkylsulfinylalkoxy-, Alkylsulfonylalkoxy-, Alkoxyalkylthio-, Alkoxyalkylsulfinyl-, Alkoxyalkylsulfonyl-, Alkylthioalkyl-, Alkylsulfinylalkyl-, Alkylsulfonylalkyl oder Dialkylaminoalkoxy-Substituenten $R^3$ oder solche Substituenten der Phenylgruppe des Restes $R^2$ besitzen vorzugsweise 2 - 6, insbesondere 2 - 4 oder 3 - 6 oder 3 - 4 oder aber 2 Kohlenstoffatome. Bevorzugte Alkyl- oder Alkylenteile in diesen Resten sind Methyl, Ethyl, n-Propyl, Isopropyl bzw. entsprechend Methylen, Ethylen und Propylen. Sie sind vorzugsweise endständig substituiert. Besonders bevorzugte Reste dieser Art sind Alkoxyethoxy, Alkoxymethyl, Alkylthioethoxy, Alkylsulfinylethoxy, Alkylsulfonylethoxy, Alkoxyethylthio, Alkoxyethylsulfinyl, Alkoxyethylsulfonyl, Alkylthiomethyl, Alkylsulfinylmethyl, Alkylsulfonylmethyl und 2-Dimethylaminoethoxy.

Besonders bevorzugte Reste $R^3$ und Substituenten des Phenylkerns von $R^2$ sind Difluormethoxy und Alkoxyalkoxy, vorzugsweise Alkoxyethoxy, insbesondere Methoxyethoxy und Alkoxyalkyl, vorzugsweise Alkoxymethyl, insbesondere Methoxymethyl. Insbesondere wird 2-Methoxyethoxy bevorzugt.

Eine bevorzugte Verbindung ist
4-(3-(1-Diphenylmethyl-azetidin-3-oxy)-2-hydroxypropoxy)-1H-indol-2-carbonitril, und Salze und physiologisch hydrolysierbaren Derivate davon, insbesondere mit der S-Konfiguration am $C^{(1)*}$ im Propanolaminanteil.

Die erfindungsgemäßen Verbindungen der Formel I, ihre physiologisch verträglichen Salze und Säureadditionssalze sowie deren physiologisch hydrolysierbaren Derivate sind therapeutische Wirkstoffe, besitzen hohe pharmakologische Wirkung und sind wertvolle Arzneimittel. Insbesondere zeigen sie positiv inotrope, vasodilatierende und antiarrhythmische Wirkung und eignen sich zur Behandlung von Herzinsuffizienz, Herzrhythmusstörungen und Hypertonie, koronaren Herzerkrankungen, peripheren und zentralen arteriellen Durchblutungsstörungen.

Die Verbindungen der vorliegenden Erfindung können beim Menschen oral oder parenteral in einer Dosierung von 1 - 800 mg, vorzugsweise 10 - 200 mg, besonders bevorzugt 20 - 100 mg pro Tag, angewendet werden, insbesondere in unterteilten Dosen, zum Beispiel dreimal täglich. Diese Dosierungen sind vorteilhaft für die Behandlung der vorstehend genannten Krankheiten, insbesondere von Herzinsuffizienz und/oder Hypertonie und Arrhythmien.

Die positiv inotrope Wirkung der erfindungsgemäßen Verbindungen wurde am Meerschweinchen-Papillarmuskel bestimmt (Naunyn-Schmiedeberg's Arch. Pharmacol. 304, 37, 1978). Die Konzentration der Substanz im Organbad betrug jeweils $10^{-4}$ mol/l. Die maximale prozentuale Steigerung der Kontraktionsamplitude wurde jeweils an drei Papillarmuskeln bestimmt und betrug mindestens 50%.

Gemäß der Erfindung werden pharmazeutische Zusammensetzungen geschaffen, die eine Verbindung der Formel I oder die unter physiologischen Bedingungen zu den erfindungsgemäßen Verbindungen hydrolysierbaren Derivate, wie z.B. Ester oder deren pharmazeutisch verträgliche Salze, zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können oral in Form von Tabletten, Dragees, Sirups, Suspensionen und Flüssigkeiten oder parenteral in Form von Lösungen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurfurylalkohols und Wasser.

Die Tabletten können nach bekannten Arbeitsweisen überzogen werden, um den Zerfall und die Resorption im Magen-Darmtrakt zu verzögern, wodurch die Aktivität des Wirkstoffs sich über eine längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyoxyethylenstearat und Polyoxyethylensorbitanmonooleat und Konservierungsmitteln wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert. Die pharmazeutischen Präparate können den Wirkstoff in einer Menge von 0,1 bis 90%, insbesondere 1 bis 90%, enthalten, wobei der Rest ein Trägerstoff oder Zusatzstoff ist. Im Hinblick auf die Herstellung und Verabreichung werden feste Präparate wie Tabletten und Kapseln bevorzugt. Vorzugsweise

enthalten die Präparate den Wirkstoff in einer Menge von 5 - 50 mg.

Die Verbindungen der Formel I, können hergestellt werden durch Umsetzung der bekannten Indolderivate der Formel IX

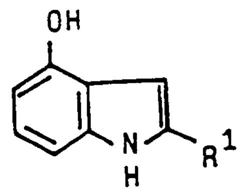

(IX)

mit Verbindungen der Formel X

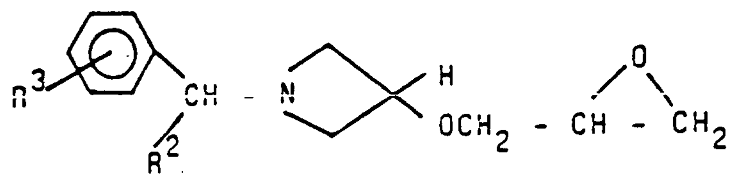

(X)

wobei $R^1$, $R^2$ und $R^3$ die obengenannte Bedeutung haben. Die Umsetzungen können in wäßrigem Dioxan oder in anderen geeigneten Lösungsmitteln in Gegenwart von Alkali, vorzugsweise Natronlauge durchgeführt werden. Die Reaktionstemperatur kann zwischen Raumtemperatur und der Siedetemperatur des Gemisches liegen. Sie liegt vorzugsweise zwischen 50 und 70°C.

Die Verbindungen der Formel X, in der $R^2$ und $R^3$ die obengenannte Bedeutung haben, können hergestellt werden durch Umsetzung von bekannten oder nach bekannten Verfahren herstellbaren Verbindungen der Formel XI, in der $R^2$ und $R^3$ die obengenannte Bedeutung haben,

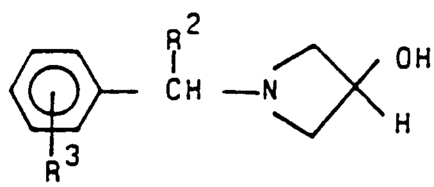

(XI)

mit Epichlorhydrin in einem geeigneten Lösungsmittel wie z.B. Dimethylsulfoxid in Gegenwart von Alkali, vorzugsweise Natronlauge bei Temperaturen zwischen 0°C bis 50°C, vorzugsweise Raumtemperatur.

Die Verbindungen der allgemeinen Formel XI können hergestellt werden durch Umsetzung von Benzhydrylaminen der allgemeinen Formel XII

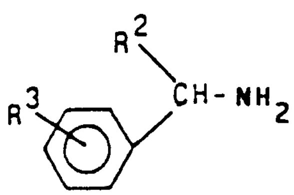

(XII)

in der $R^2$ und $R^3$ die angegebene Bedeutung haben, mit Epichlorhydrin in einem geeigneten Lösungsmittel wie Methanol, vorzugsweise bei Raumtemperatur bis Siedetemperatur des Gemisches, insbesondere bei 70°C.

Die Verbindungen der Formel XII können nach an sich bekannten Verfahren bzw. analog zu den hier beschriebenen oder analog zu an sich bekannten Verfahren hergestellt werden (Literatur: Beilstein, viertes Ergänzungswerk 12, Systemnummer 1734 und Najer et al., Bl. 1959, 352 - 355).

Eine gegebenenfalls erforderliche Veresterung der Hydroxygruppe in der 3-Amino-propoxy-Seitenkette kann analog zu für die Herstellung analoger Ester von 3-Amino-2-hydroxypropoxyaryl-Verbindungen bekannten Methoden durchgeführt werden, nötigenfalls unter selektiven Bedingungen, falls andere reaktionsfähige Gruppen vorliegen.

Die verwendeten Ausgangsmaterialien sind bekannt oder können nach an sich bekannten Verfahren bzw. analog zu den hier beschriebenen oder analog zu an sich bekannten Verfahren hergestellt werden.

Die Verbindungen der allgemeinen Formel I können sowohl Basen als auch Säuren beziehungsweise amphoter sein und daher in der Form ihrer Salze oder Säureadditionssalze aus den Reaktionsgemischen isoliert werden. Sie lassen sich als Basen mit geeigneten anorganischen oder organischen Säuren nach bekannten Verfahren in Salze überführen oder bilden als Säuren mit Basen Salze.

Bevorzugt werden physiologisch verträgliche Salze oder Säureadditionssalze. Hierfür sind als anorganische Säuren beispielsweise Schwefelsäure oder Halogenwasserstoffsäuren, zum Beispiel Salzsäure, und als organische Säuren, zum Beispiel Fumarsäure, Maleinsäure, Zitronensäure und Weinsäure, geeignet. Zur Herstellung wird die heiße alkoholische Lösung der Base mit der alkoholischen Lösung einer geeigneten Säure versetzt, und man erhält nach Etherzusatz das Salz. Bevorzugte Salze sind die Alkali-, Erdalkali- und Ammoniumsalze der Verbindungen der Formel I, die mit den entsprechenden Basen, insbesondere Natrium-, oder Kaliumhydroxid erhalten werden.

Die erfindungsgemäßen Verbindungen der Formel I weisen am Kohlenstoffatom 2 der Propoxyseitenkette ein Chiralitätszentrum auf und können, abhängig von den Substituenten, weitere asymmetrische Kohlenstoffatome besitzen und daher als Racemate und Diastereoisomere vorliegen. Diastereoisomere können in bekannter Weise aufgrund der physikalisch-chemischen Unterschiede ihrer Bestandteile in ihre racemischen Modifikationen getrennt werden. Racemate können nach bekannten Methoden getrennt werden, beispielsweise durch Umkristallisieren in optisch aktiven Lösungsmitteln, durch Mikroorganismen oder Reaktion mit einer optisch aktiven Säure oder Base, die mit der racemischen Verbindung ein Salz bildet, Trennung der Diastereoisomeren durch fraktionierte Kristallisation und Freisetzung der Enantiomeren durch geeignete Mittel. Besonders geeignete optisch aktive Säuren sind beispielsweise die d- und l-Formen der Weinsäure, Ditoluylweinsäure, Äpfelsäure, Mandelsäure, Kamphersulfonsäure oder Pyrrolidon-carbonsäure. Geeignete optisch aktive Basen sind alpha-Phenyläthylamin, Menthylamin, Ephedrin, Brucin und Chinin. Vorteilhafterweise wird der aktivere der Antipoden isoliert. Gemäß der Erfindung ist es jedoch auch möglich, die reinen Enantiomeren durch asymmetrische Synthese zu erhalten.

Die folgenden Beispiele dienen zur Erläuterung der Erfindung.

Herstellungsbeispiel

1-(Diphenylmethyl)-3-(2,3-epoxypropoxy)-azetidin

37,5 g Diphenylmethyl-azetidin-3-ol werden bei Raumtemperatur in einem Gemisch aus 250 ml Dimethylsulfoxid und 150 ml 5%iger Natronlauge gelöst, mit 65 ml Epichlorhydrin versetzt und 3 Tage bei Raumtemperatur stehen gelassen. Das Reaktionsgemisch wird mit 300 ml Methylenchlorid extrahiert, die organische Phase über Natriumsulfat getrocknet und eingedampft. Das Rohprodukt wird im Vakuum destilliert.
Ausbeute: 29.0 g
1-(Diphenylmethyl)-3-(2,3-epoxypropoxy)-azetidin
Kp. 174 - 176°C 0.2 mbar

Beispiel 1

4-(3-(1-Diphenylmethyl-azetidin-3-oxy)-2-hydroxypropoxy)-1H-indol-2-carboxamid

8,8 g 4-Hydroxy-1H-indol-2-carboxamid werden in 250 ml 0.8 prozentiger Natronlauge gelöst und mit einer Lösung von 16,3 g 1-(Diphenylmethyl)-3-(2,3-epoxypropoxy)-azetidin in 250 ml Dioxan versetzt. Man läßt das Gemisch 4 Stunden bei Raumtemperatur stehen und erwärmt dann weitere 20 Stunden auf 60°C. Das Reaktionsgemisch wird dann in 1 l Eiswasser eingerührt und zweimal mit je 500 ml Methylenchlorid extrahiert. Man trocknet die organische Phase mit Kaliumcarbonat und destilliert das Lösungsmittel im Vakuum ab. Der Rückstand wird zweimal mit je 100 ml Diisopropylether verrieben und abgesaugt.
Ausbeute: 7.1 g
4-(3-(1-Diphenylmethyl-azetidin-3-oxy)-2-hydroxypropxy)-1H-indol-2-carboxamid
Schmp. 186°C

Beispiel 2

4-(3-(1-Diphenylmethyl-azetidin-3-oxy)-2-hydroxypropoxy)-1H-indol-2-carbonitril

10,0 g 4-(3-(1-Diphenylmethyl-azetidin-3-oxy)-2-hydroxypropoxy)-1H-indol-2-carboxamid werden in einem Gemisch aus 112 ml Dioxan und 10 g Pyridin gelöst und bei 10°C mit 10 ml Trifluoracetanhydrid in 10 ml Dioxan versetzt. Nach zweistündigem Stehen bei Raumtemperatur wird das Gemisch in Eiswasser eingerührt. Man extrahiert mit Methylenchlorid, wäscht erst mit verdünnter NaOH, dann mit Wasser, trocknet die organische Phase über Natriumsulfat und engt ein. Der Rückstand wird säulenchromatographisch an Kieselgel gereinigt (Laufmittel: Chloroform).
Ausbeute: 9,3 g (41%) 4-(3-(1-Diphenylmethyl-azetidin-3-oxy)-2-hydroxypropoxy)-1H-indol-2-carbonitril
Schmp. : 83 - 85°C

Beispiel 3

Herstellung von Ampullen

Ampullen, die die im folgenden genannten Bestandteile enthalten, lassen sich in bekannter Weise herstellen. Der Wirkstoff wird in Wasser und 1,2-Propandiol gelöst und unter Stickstoff in Glasampullen abgefüllt.

| | |
|---|---|
| 4-(3-(1-Diphenylmethyl-azetidin-3-oxy)-2-hydroxypropoxy)-1H-indol-2-carbonitril | 2 mg |
| 1,2-Propandiol | 0,8 ml |
| dest. Wasser ad | 2,0 ml |

Beispiel 4

Herstellung von Tabletten und Kapseln

Tabletten und Kapseln, welche die nachstehend angegebenen Bestandteile enthalten, werden nach bekannten Arbeitsweisen hergestellt. Diese sind für die Behandlung der vorstehend genannten Krankheiten, insbesondere der Herzinsuffizienz in Dosierungsmengen von jeweils einer Tablette oder Kapsel dreimal täglich geeignet.

| Bestandteile | Gewicht (mg) | |
|---|---|---|
| | Tablette | Kapsel |
| 4-(3-(1-Diphenylmethyl-azetidin-3-oxy)-2-hydroxypropoxy)-1H-indol-2-carbonitril | 30 | 10 |
| Tragacanth | 10 | |
| Lactose | 247,5 | 300 |
| Maisstärke | 25 | |
| Talk | 15 | |
| Magnesiumstearat | 2,5 | |

Analog zu den vorstehenden Beispielen 1 und 2 können die in der folgenden Tabelle angegebenen erfindungsgemäßen Verbindungen der Formel I erhalten werden, (Beispiele 1 - 11):

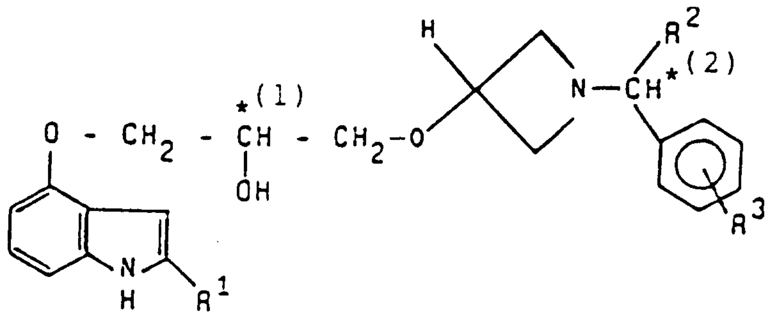

"rac" bedeutet racemisch. Soweit nicht durch $R^4$ anders angegeben, liegt die 2-Hydroxygruppe der Propoxyamino-Seitenkette in nichtveresterter Form vor.
"---" bedeutet, daß kein asymmetrisches Kohlenstoffatom vorliegt.

| Beispiel | $R^1$ | $R^2$ | $R^3$ | Konfig. $C^*(1)$ | Konfig. $C^*(2)$ | Schmp. °C |
|---|---|---|---|---|---|---|
| 3 | CN | Phenyl | 3-$OCH_3$ | rac | rac | |
| 4 | CN | Phenyl | 2-Cl | rac | rac | |
| 5 | CN | 4-Cl-Phenyl | 4-Cl | rac | — — — | |
| 6 | CN | Phenyl | 4-Cl | rac | rac | |
| 7 | CN | Phenyl | 4-$OCH_3$ | rac | rac | |
| 8 | $CONH_2$ | Phenyl | H | rac | — — — | 186 |
| 9 | CN | 4-F-Phenyl | 4-F | rac | — — — | 78 - 80 |
| 10 | CN | Phenyl | H | S | — — — | 79 |
| 11 | CN | Phenyl | O-$(CH_2)_2OCH_3$ | rac | rac | |

**Patentansprüche**

**Patentansprüche für folgende Vertragsstaaten : AT, BE, CH, DE, FR, GB, IT, LI, NL, SE**

1. Substituierte Indolylpropanole der Formel I

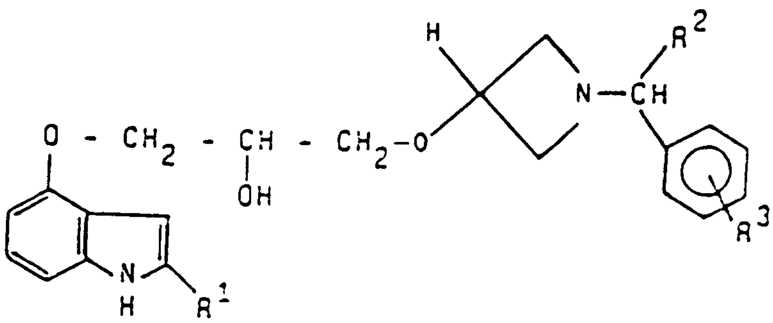

in der
$R^1$ eine Cyano-, Carboxamid-, Alkoxycarbonyl-, Hydroxyl- oder Acetylgruppe bedeutet,
$R^2$ Pyridinyl, Thienyl, Phenyl oder substituiertes Phenyl bedeutet, das durch Halogen, Alkyl, Cycloalkyl, Alkoxy, Difluormethoxy, Difluormethylthio, Trifluormethoxy, Trifluormethylthio, Trifluorethoxy, Alkoxyalkoxy, Cycloalkylalkoxy, Alkoxyalkyl, Alkylthioalkoxy, Alkylsulfinylalkoxy, Alkylsulfonylalkoxy, Alkoxyalkylthio, Alkoxyalkylsulfinyl, Alkoxyalkylsulfonyl, Alkylthioalkyl, Alkylsulfinylalkyl, Alkylsulfonylalkyl und Dialkylaminoalkoxy mono- oder disubstituiert ist, und
$R^3$ Wasserstoff, Alkoxy, Halogen, Difluormethoxy, Difluormethylthio, Trifluormethoxy, Trifluormethylthio, Trifluorethoxy, Alkoxyalkoxy, Alkoxyalkyl, Alkylthioalkoxy, Alkylsulfinylalkoxy, Alkylsulfonylalkoxy, Alkoxyalkylthio, Alkoxyalkylsulfinyl, Alkoxyalkylsulfonyl, Alkylthioalkyl, Alkylsulfinylalkyl, Alkylsulfonylalkyl oder Dialkylaminoalkoxy bedeutet,
oder ein physiologisch verträgliches hydrolysierbares Derivat davon, in dem die Hydroxygruppe in 2-Stellung der 3-Aminopropoxy-Seitenkette in veresterter Form vorliegt, sowie ihre tautomeren Formen und ihre Salze sowie Säureadditionssalze, wobei in allen vorstehend genannten Fällen Alkyl der Alkylgruppen oder von Alkylteilen oder Alkylenteilen von Gruppen jeweils geradkettiges oder verzweigtes Alkyl beziehungsweise Alkylen mit 1 bis 6 Kohlenstoffatomen bedeutet und Cycloalkyl 3 bis 7 Kohlenstoffatome besitzt.

2. 4-(3-(1-Diphenylmethyl-azetidin-3-oxy)-2-hydroxypropoxy)-1H-indol-2-carbonitril

3. Verfahren zur Herstellung von Verbindungen der Formel I, gemäß Anspruch 1 in der $R^1$ eine Cyano- bzw. Nitrilgruppe bedeutet, dadurch gekennzeichnet, daß man entsprechende Verbindungen der Formel I, in der $R^1$ eine Carboxamidogruppe bedeutet, dehydratisiert.

4. Verfahren zur Herstellung der Verbindungen der Formel I, gemäß Anspruch 1 dadurch gekennzeichnet, daß man Indolderivate der Formel IX

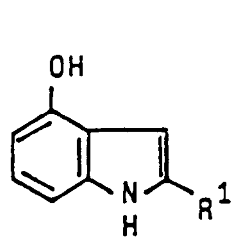

( IX )

in der $R^1$ die oben angegebene Bedeutung hat, mit Verbindungen der Formel X

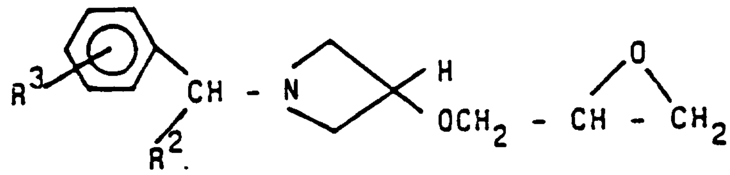 (X)

in der $R^2$ und $R^3$ die angegebene Bedeutung haben, umsetzt, und die erhaltenen Verbindungen gegebenenfalls verestert und in Salze überführt.

5. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

6. Verbindung gemäß Anspruch 1 als pharmazeutischer Wirkstoff, insbesondere zur Behandlung von Herzinsuffizienz, Herzrhythmusstörungen und Hypertonie, koronaren Herzerkrankungen, peripheren und zentralen arteriellen Durchblutungsstörungen.

**Patentansprüche für folgenden Vertragsstaat : ES**

1. Verfahren zur Herstellung der Verbindungen der Formel I,

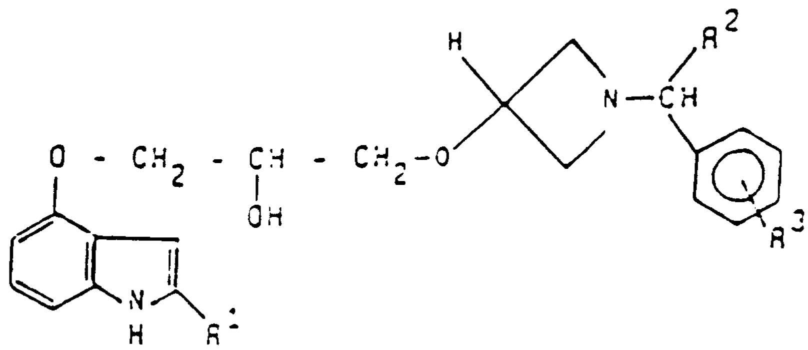

in der
$R^1$ eine Cyano-, Carboxamid-, Alkoxycarbonyl-, Hydroxyl- oder Acetylgruppe bedeutet,
$R^2$ Pyridinyl, Thienyl, Phenyl oder substituiertes Phenyl bedeutet, das durch Halogen, Alkyl, Cycloalkyl, Alkoxy, Difluormethoxy, Difluoremethylthio, Trifluormethoxy, Trifluormethylthio, Trifluorethoxy, Alkoxyalkoxy, Cycloalkylalkoxy, Alkoxyalkyl, Alkylthioalkoxy, Alkylsulfinylalkoxy, Alkylsulfonylalkoxy, Alkoxyalkylthio, Alkoxyalkylsulfinyl, Alkoxyalkylsulfonyl, Alkylthioalkyl, Alkylsulfinylalkyl, Alkylsulfonylalkyl und Dialkylaminoalkoxy mono- oder disubstituiert ist, und
$R^3$ Wasserstoff, Alkoxy, Halogen, Difluormethoxy, Difluormethylthio, Trifluormethoxy, Trifluormethylthio, Trifluorethoxy, Alkoxyalkoxy, Alkoxyalkyl, Alkylthioalkoxy, Alkylsulfinylalkoxy, Alkylsulfonylalkoxy, Alkoxyalkylthio, Alkoxyalkylsulfinyl, Alkoxyalkylsulfonyl, Alkylthioalkyl, Alkylsulfinylalkyl, Alkylsulfonylalkyl oder Dialkylaminoalkoxy bedeutet,
oder ein physiologisch vertragliches hydrolysierbares Derivat davon, in dem die Hydroxygruppe in 2-Stellung der 3-Aminopropoxy-Seitenkette in veresterter Form vorliegt, sowie ihre tautomeren formen und ihre Salze sowie Säureadditionssalze, wobei in allen vorstehend genannten Fällen Alkyl der Alkylgruppen oder von Alkylteilen oder Alkylenteilen von Gruppen jeweils geradkettiges oder verzweigtes Alkyl beziehungsweise Alkylen mit 1 bis 6 Kohlenstoffatomen bedeutet und Cycloalkyl 3 bis 7 Kohlenstoffatome besitzt, dadurch gekennzeichnet, daß man Indolderivate der Formel IX

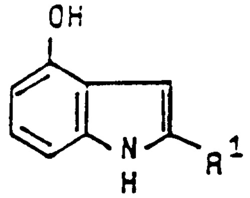

(IX)

in der $R^1$ die oben angegebene Bedeutung hat, mit Verbindungen der Formel X

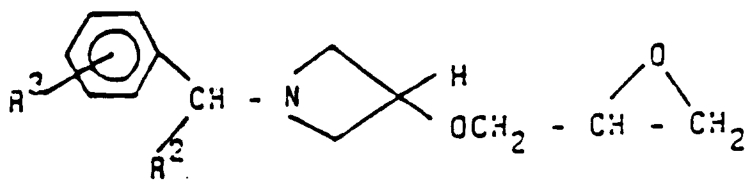

(X)

in der $R^2$ und $R^3$ die angegebene Bedeutung haben, umsetzt, und die erhaltenen Verbindungen gegebenenfalls verestert und in Salze überführt.

**2.** Verfahren zur Herstellung von Verbindungen der Formel I, gemäß Anspruch 1, in der $R^1$ eine Cyano- bzw. Nitrilgruppe bedeutet, dadurch gekennzeichnet, daß man entsprechende Verbindungen der Formel I, in der $R^1$ eine Carboxamidogruppe bedeutet, dehydratisiert.

**3.** Verfahren zur Herstellung gemäß Anspruch 2 von 4-(3-(1-Diphenylmethyl-azetidin-3-oxy)-2-hydroxypropoxy)-1H-indol-2-carbonitril.

## Claims

**Claims for the following Contracting States : AT, BE, CH, DE, FR, GB, IT, LI, NL, SE**

**1.** Substituted indolylpropanols of the formula I

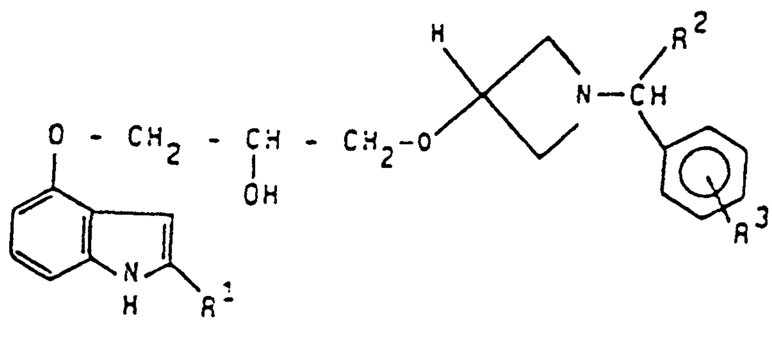

in which

- $R^1$ is a cyano, carboxamide, alkoxycarbonyl, hydroxyl or acetyl group,
- $R^2$ is pyridinyl, thienyl, phenyl or substituted phenyl, which is mono- or disubstituted by halogen, alkyl, cycloalkyl, alkoxy, difluoromethoxy, difluoromethylthio, trifluoromethoxy, trifluoromethylthio, trifluoroethoxy, alkoxyalkoxy, cycloalkylalkoxy, alkoxyalkyl, alkylthioalkoxy, alkylsulphinylalkoxy, alkylsulphonylalkoxy, alkoxyalkylthio, alkoxyalkylsulphinyl, alkoxyalkylsulphonyl, alkylthioalkyl, alkylsulphinylalkyl, alkylsulphonylalkyl and dialkylaminoalkoxy, and
- $R^3$ is hydrogen, alkoxy, halogen, difluoromethoxy, difluoromethylthio, trifluoromethoxy, trifluoromethylthio, trifluoroethoxy, alkoxyalkoxy, alkoxyalkyl, alkylthioalkoxy, alkylsulphinylalkoxy, alkylsulphonylalkoxy, alkoxyalkylthio, alkoxyalkylsulphinyl, alkoxyalkylsulphonyl, alkylthioalkyl, alkylsulphinylalkyl, alkylsulphonylalkyl or dialkylaminoalkoxy,

or a physiologically tolerated hydrolysable derivative thereof in which the hydroxyl group in

the 2-position of the 3-aminopropoxy side chain is present in esterified form, and their tautomeric forms and their salts and acid addition salts, wherein, in all the abovementioned cases, alkyl of alkyl groups or of alkyl moieties or alkylene moieties of groups in each case is straight-chain or branched alkyl or alkylene having 1 to 6 carbon atoms and cycloalkyl has 3 to 7 carbon atoms.

**2.** 4-(3-(1-Diphenylmethyl-azetidin-3-oxy)-2-hydroxypropoxy)-1H-indole-2-carbonitrile.

**3.** Process for the preparation of compounds of the formula I according to Claim 1, in which $R^1$ is a cyano or nitrile group, characterized in that corresponding compounds of the formula I in which $R^1$ is a carboxamido group are dehydrated.

**4.** Process for the preparation of compounds of the formula I according to Claim 1, characterized in that indole derivatives of the formula IX

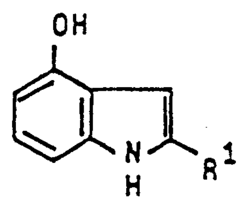

(IX)

in which $R^1$ has the abovementioned meaning, are reacted with compounds of the formula X

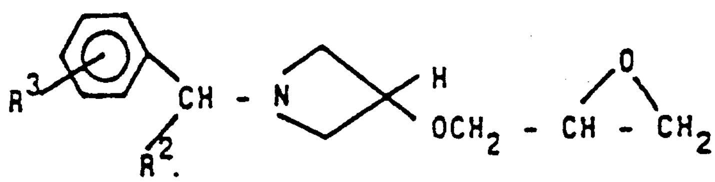

(X)

in which $R^2$ and $R^3$ have the meaning given, and, if appropriate, the resulting compounds are esterified and converted into salts.

**5.** Pharmaceutical preparation, characterized in that it comprises one or more of the compounds according to Claim 1 or physiologically tolerated salts thereof and if appropriate customary carrier substances and/or diluents.

**6.** Compound according to Claim 1 as a pharmaceutical active compound, in particular for the treatment of cardiac insufficiency, disturbances in cardiac rhythm and hypertension, coronary heart diseases and disturbances in peripheral and central arterial circulation.

**Claims for the following Contracting State : ES**

**1.** Process for the preparation of the compounds of the formula I

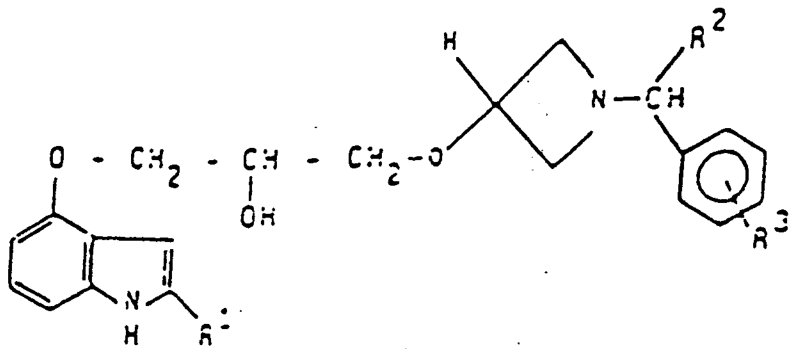

in which

$R^1$ is a cyano, carboxamide, alkoxycarbonyl, hydroxyl or acetyl group,

$R^2$ is pyridinyl, thienyl, phenyl or substituted phenyl, which is mono- or disubstituted by halogen, alkyl, cycloalkyl, alkoxy, difluoromethoxy, difluoromethylthio, trifluoromethoxy, trifluoromethylthio, trifluoroethoxy, alkoxyalkoxy, cycloalkylalkoxy, alkoxyalkyl, alkylthioalkoxy, alkylsulphinylalkoxy, alkylsulphonylalkoxy, alkoxyalkylthio, alkoxyalkylsulphinyl, alkoxyalkylsulphonyl, alkylthioalkyl, alkylsulphinylalkyl, alkylsulphonylalkyl and dialkylaminoalkoxy, and

$R^3$ is hydrogen, alkoxy, halogen, difluoromethoxy, difluoromethylthio, trifluoromethoxy, trifluoromethylthio, trifluoroethoxy, alkoxyalkoxy, alkoxyalkyl, alkylthioalkoxy, alkylsulphinylalkoxy, alkylsulphonylalkoxy, alkoxyalkylthio, alkoxyalkylsulphinyl, alkoxyalkylsulphonyl, alkylthioalkyl, alkylsulphinylalkyl, alkylsulphonylalkyl or dialkylaminoalkoxy,

or a physiologically tolerated hydrolysable derivative thereof in which the hydroxyl group in the 2-position of the 3-aminopropoxy side chain is present in esterified form, and their tautomeric forms and their salts and acid addition salts, wherein, in all the abovementioned cases, alkyl of alkyl groups or of alkyl moieties or alkylene moieties of groups in each case is straight-chain or branched alkyl or alkylene having 1 to 6 carbon atoms and cycloalkyl has 3 to 7 carbon atoms, characterized in that indole derivatives of the formula IX

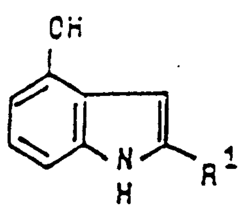

( IX )

in which $R^1$ has the abovementioned meaning, are reacted with compounds of the formula X

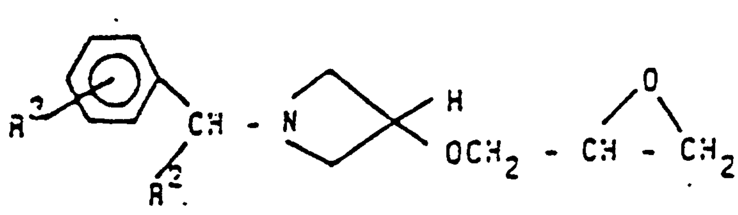

( X )

in which $R^2$ and $R^3$ have the meaning given, and, if appropriate, the resulting compounds are

esterified and converted into salts.

**2.** Process for the preparation of compounds of the formula I according to Claim 1, in which $R^1$ is a cyano or nitrile group, characterized in that corresponding compounds of the formula I in which $R^1$ is a carboxamido group are dehydrated.

**3.** Process for the preparation, according to Claim 2, of 4-(3-(1-diphenylmethyl-azetidin-3-oxy)-2-hydroxypropoxy)-1H-indole-2-carbonitrile.

## Revendications
**Revendications pour les Etats contractants suivants : AT, BE, CH, DE, FR, GB, IT, LI, NL, SE**

**1.** Indolylpropanols substitués de formule I

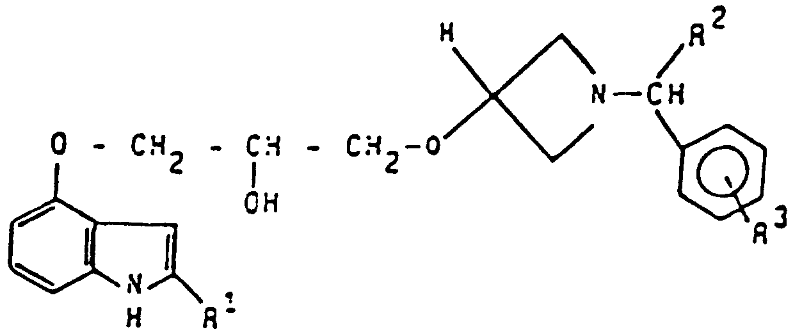

dans laquelle

$R^1$ représente un groupe cyano, un groupe carboxamide, un groupe alcoxycarbonyle, un groupe hydroxyle ou un groupe acétyle,

$R^2$ représente un groupe pyridinyle, un groupe thiényle, un groupe phényle ou un groupe phényle substitué qui peut être mono- ou disubstitué par un atome d'halogène, par un groupe alkyle, par un groupe cycloalkyle, par un groupe alcoxy, par un groupe difluorométhoxy, par un groupe difluorométhylthio, par un groupe trifluorométhoxy, par un groupe trifluorométhylthio, par un groupe trifluoréthoxy, par un groupe alcoxyalcoxy, par un groupe cycloalkylalcoxy, par un groupe alcoxyalkyle, par un groupe alkylthioalcoxy, par un groupe alkylsulfinylalcoxy, par un groupe alkylsulfonylalcoxy, par un groupe alcoxyalkylthio, par un groupe alcoxyalkylsulfinyle, par un groupe alcoxyalkylsulfonyle, par un groupe alkylthioalkyle, par un groupe alkylsulfinylalkyle, par un groupe alkylsulfonylalkyle et par un groupe dialkylaminoalcoxy, et

$R^3$ représente un atome d'hydrogène, un groupe alcoxy, un atome d'halogène, un groupe difluorométhoxy, un groupe difluorométhylthio, un groupe trifluorométhoxy, un groupe trifluorométhylthio, un groupe trifluoréthoxy, un groupe alcoxyalcoxy, un groupe alcoxyalkyle, un groupe alkylthioalcoxy, un groupe alkylsulfinylalcoxy, un groupe alkylsulfonylalcoxy, un groupe alcoxyalkylthio, un groupe alcoxyalkylsulfinyle, un groupe alcoxyalkylsulfonyle, un groupe alkylthioalkyle, un groupe alkylsulfinylalkyle, un groupe alkylsulfonylalkyle ou un groupe dialkylaminoalcoxy, ou encore un de ses dérivés hydrolysables physiologiquement acceptables, dans lequel le groupe hydroxyle en position 2 de la chaîne latérale du groupe 3-aminopropoxy est présent sous forme estérifiée, ainsi que leurs formes tautomères et leurs sels, de même que leurs sels d'addition d'acides, dans lesquels, dans tous les cas mentionnés ci-dessus, le terme alkyle des groupes alkyle ou encore des fractions alkyle ou des fractions alkylène de groupes désigne chaque fois un groupe alkyle, respectivement un groupe alkylène à chaîne droite ou ramifiée contenant de 1 à 6 atomes de carbone, et un groupe cycloalkyle contient de 3 à 7 atomes de carbone.

**2.** 4-(3-(1-diphénylméthyl-azétidin-3-oxy)-2-hydroxypropoxy)-1H-indole-2-carbonitrile.

**3.** Procédé pour la préparation de composés répondant à la formule I selon la revendication 1, dans laquelle $R^1$ représente un groupe cyano ou un groupe nitrile, caractérisé en ce qu'on soumet à une déshydratation des composés correspondants de formule I, dans laquelle $R^1$ représente un groupe carboxamido.

**4.** Procédé pour la préparation des composés de formule I selon la revendication 1, caractérisé en ce qu'on fait réagir des dérivés d'indole de formule IX

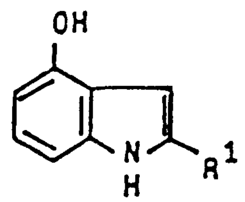

( IX )

dans laquelle $R^1$ a la signification indiquée ci-dessus, avec des composés de formule X

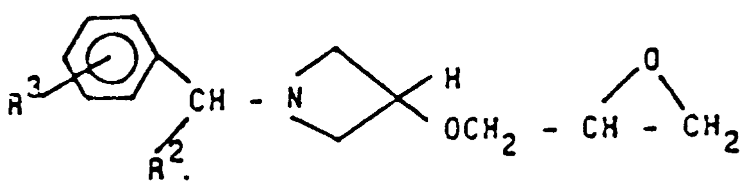

( X )

dans laquelle $R^2$ et $R^3$ ont la signification indiquée, et on estérifie éventuellement les composés obtenus et on les transforme en sels.

**5.** Préparation pharmaceutique caractérisée en ce qu'elle contient un ou plusieurs des composés selon la revendication 1 ou de leurs sels physiologiquement acceptables et éventuellement des substances de support et/ou des diluants habituels.

**6.** Composé selon la revendication 1, comme substance active pharmaceutique, en particulier pour le traitement de l'insuffisance cardiaque, des troubles du rythme cardiaque et de l'hypertonie, des maladies coronaires du coeur, des troubles de la circulation artérielle périphérique et centrale.

**Revendications pour l'Etat contractant suivant : ES**

**1.** Procédé pour la préparation des composés de formule I

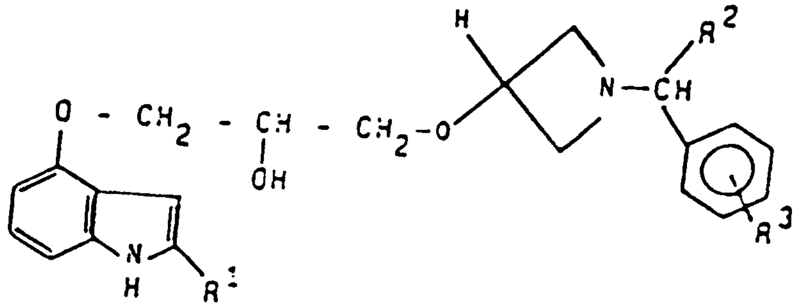

dans laquelle
$R^1$ représente un groupe cyano, un groupe carboxamide, un groupe alcoxycarbonyle, un groupe

hydroxyle ou un groupe acétyle,

$R^2$ représente un groupe pyridinyle, un groupe thiényle, un groupe phényle ou un groupe phényle substitué qui peut être mono- ou disubstitué par un atome d'halogène, par un groupe alkyle, par un groupe cycloalkyle, par un groupe alcoxy, par un groupe difluorométhoxy, par un groupe difluorométhylthio, par un groupe trifluorométhoxy, par un groupe trifluorométhylthio, par un groupe trifluoréthoxy, par un groupe alcoxyalcoxy, par un groupe cycloalkylalcoxy, par un groupe alcoxyalkyle, par un groupe alkylthioalcoxy, par un groupe alkylsulfinylalcoxy, par un groupe alkylsulfonylalcoxy, par un groupe alcoxyalkylthio, par un groupe alcoxyalkylsulfinyle, par un groupe alcoxyalkylsulfonyle, par un groupe alkylthioalkyle, par un groupe alkylsulfinylalkyle, par un groupe alkylsulfonylalkyle et par un groupe dialkylaminoalcoxy, et

$R^3$ représente un atome d'hydrogène, un groupe alcoxy, un atome d'halogène, un groupe difluorométhoxy, un groupe difluorométhylthio, un groupe trifluorométhoxy, un groupe trifluorométhylthio, un groupe trifluoréthoxy, un groupe alcoxyalcoxy, un groupe alcoxyalkyle, un groupe alkylthioalcoxy, un groupe alkylsulfinylalcoxy, un groupe alkylsulfonylalcoxy, un groupe alcoxyalkylthio, un groupe alcoxyalkylsulfinyle, un groupe alcoxyalkylsulfonyle, un groupe alkylthioalkyle, un groupe alkylsulfinylalkyle, un groupe alkylsulfonylalkyle ou un groupe dialkylaminoalcoxy, ou encore un de ses dérivés hydrolysables physiologiquement acceptables, dans lequel le groupe hydroxyle en position 2 de la chaîne latérale du groupe 3-aminopropoxy est présent sous forme estérifiée, ainsi que leurs formes tautomères et leurs sels, de même que leurs sels d'addition d'acides, dans lesquels, dans tous les cas mentionnés ci-dessus, le terme alkyle des groupes alkyle ou encore des fractions alkyle ou des fractions alkylène de groupes désigne chaque fois un groupe alkyle, respectivement un groupe alkylène à chaîne droite ou ramifiée contenant de 1 à 6 atomes de carbone, et un groupe cycloalkyle contient de 3 à 7 atomes de carbone, caractérisé en ce qu'on fait réagir des dérivés d'indole de formule IX

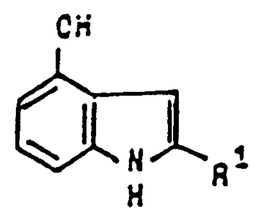

(IX)

dans laquelle $R^1$ a la signification indiquée ci-dessus, avec des composés de formule X

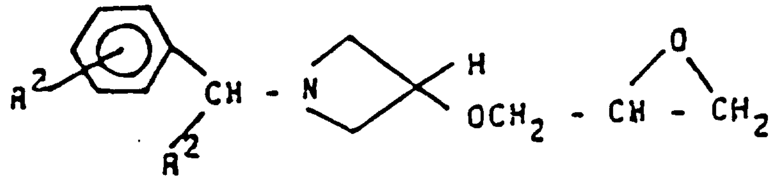

(X)

dans laquelle $R^2$ et $R^3$ ont la signification indiquée, et on estérifie éventuellement les composés obtenus et on les transforme en sels.

2. Procédé pour la préparation de composés répondant à la formule I selon la revendication 1, dans laquelle $R^1$ représente un groupe cyano ou un groupe nitrile, caractérisé en ce qu'on soumet à une déshydratation des composés correspondants de formule I, dans laquelle $R^1$ représente un groupe carboxamido.

3. Procédé pour la préparation selon la revendication 2, du 4-(3-(1-diphénylméthyl-azétidin-3-oxy)-2-hydroxypropoxy)-1H-indole-2-carbonitrile.